# EUROPEAN PATENT SPECIFICATION

(11) **EP 1 832 255 B1**
(45) Date of publication and mention of the grant of the patent: **08.09.2010**
(21) Application number: 06425158.0
(22) Date of filing: 09.03.2006
(51) Int. Cl.: A61F 5/01

(54) **Knee brace articulated joint with quick-release restraining means**
Scharniergelenk für eine Knieschiene mit Schnellverschluss
Articulation pour genouillère avec mécanisme de fixation rapide

(43) Date of publication of application: 12.09.2007
(73) Proprietor: F.G.P. Srl, 37062 Dossobuono (VR) (IT)
(72) Inventor: Turrini, Alberto, 37062 Castel D'Azzano (VR) (IT); Ferrigolo, Moreno, 37062 Dossobuono (VR) (IT)
(74) Representative: Petraz, Gilberto Luigi

(56) References cited:
- GB-A- 2 326 098
- US-A- 5 460 599
- US-A- 5 921 946

## Description

### TECHNICAL FIELD

This invention concerns a knee brace articulated joint equipped with quick-release restraining means.

More specifically, this is an articulated joint equipped with a quick-release device that allows a knee brace to be held in the blocked position while being used for walking and to be quickly released by the user when necessary, for example to allow the user to sit down or to make other movements which involve partial flexing of the knee joint in passive conditions or in any case not while in weight-bearing conditions.

The invention in question is particularly useful in resolving the problem of a quick release in the situations described above.

This invention can be applied in the industrial field relative to the production of equipment for orthopedic use and in particular orthopedic equipment or braces designed to maintain the knee joint in an immobilised condition.

### BACKGROUND ART

It is known that for some disorders of an orthopedic nature, particularly those involving the knee, the elbow or the ankle, as a result injuries to ligaments or other parts of these joints or following surgical operations, it is necessary to use a knee brace or other form of support for legs or arms designed to guarantee a hinged restraint function, for example between the femur and the tibia, withstanding most of the strain which otherwise would be harmful for the rehabilitation of the knee.

Considering that similar conditions also apply to other types of brace, for example for the elbow, the ankle and other parts of the leg or arm, the traditional knee brace usually consists of a rigid frame enclosing the limb at the level of the joint, able to protect the joint from all the strain that would be extremely harmful for the injured and/or convalescent subject during walking.

The frame of a knee brace or other similar type of brace generally comprises means for restraining the brace to the femur and the tibia close to the knee and a structure connecting these means consisting of an articulated joint positioned at the level of the knee.

According to background art, knee brace articulated joints usually consist of a pair of discs hinged to each other, each one being integral with a respective upright fixed in turn to the wearer's limb.

Since it is indispensable in post-traumatic treatment for the angular excursion between one disc and the other to be limited within a range gradually increasing in relation to time, and that the control of angular excursion during extension must be different to the control during flexion, the articulated joint must be equipped with easily accessible and easy to operate adjustment devices.

It has been found that the adjustment of these orthopedic brace joints creates some difficulty both for specialised orthopedic personnel and, according to specific information provided by general practitioners, for the user if he/she is able to intervene directly on the brace being worn.

All the solutions described so far which foresee the use of orthopedic braces equipped with articulated joints, in particular knee braces, are however limited in terms of practicality as they do not allow the knee brace to be maintained in a blocked position when used in weight-bearing conditions and when necessary to be quickly released by the user, for example to allow the user to sit down or to make other movements that involve partial flexing of the knee joint even in passive or non-weight-bearing conditions.

The quick deactivation of the angular excursion devices, for example by slightly flexing the limb in non-weight-bearing conditions, is not therefore foreseen in traditional systems and the variation of the range entrusted to reference elements also means that the device is difficult for the user to manage, having to reset the load angles each time.

US-A-5921946 discloses a hinge for orthopedic and rehabilitation braces provided with a flexion and an extension switch allowing a repositioning of stops or limits which limit the flexion and extension of the brace. These switches may be repositioned, however, only with repositioning pressure for moving the switch to a new position combined with safety pressure for unlocking the switch. A brace locking switch may also be included.

GB-A-2326098 discloses a hinge for a lockable knee brace comprising first and second hinge members rotatably connected together and means for locking the two hinge members together in a selected orientation. The locking means comprises a pivotal lever mounted on the hinge member having a latch to be received in a slot and movable between a locking position and an unlocking position and being retained in both positions by means of a retaining mechanism on the lever.

US-A-5460599 discloses an orthopedic hinge assembly for connecting an upper and lower portion of a brace, including a housing member with an abductor member movably mounted on the housing member. A pair of adjuster members are also mounted to the housing member and a stop member attached to the abductor member extends through arcuate apertures on the adjuster members.

### DESCRIPTION OF THE INVENTION

This invention proposes to provide a knee brace articulated joint equipped with quick release means that is able to overcome the problems described above.

The solution proposed by this invention does in fact allow the user to release the orthopedic brace when it is necessary to slightly flex the joint passively, for example when sitting down or getting into cars or in other situations which require slight flexing of the joint in non-weight-bearing conditions.

The invention also proposes to provide a knee brace articulated joint equipped with quick release means that is easy to produce in order to be economically advantageous.

This is achieved by means of a knee brace articulated joint equipped with quick release means with the features described in the main claim.

The dependent claims described advantageous embodiments of the invention.

The knee brace articulated joint equipped with quick release means according to the invention is therefore produced by using a cursor which wedges into the mechanism of the joint so as to function in the classic way with the angular excursion between one disc and the other limited within a range that gradually increases in relation to time

If the user wishes to flex the joint freely, for example when sitting down or remaining in a passive flexion condition, to release the end-of-stroke between one disc and the other it is sufficient to slightly push the safety tab upwards, for example, so that the two discs are completely free and unrestrained to make any movement in a passive condition, that is to say when no weight is placed on the leg.

In this way the device can be used in all resting situations in which the knee joint is totally free even though restrained by the brace which remains firmly attached to the leg.

### DESCRIPTION OF THE DRAWINGS

Other features and advantages of the invention will become evident on reading the following description of one embodiment of the invention, given as a non-binding example, together with the accompanying drawings in which:

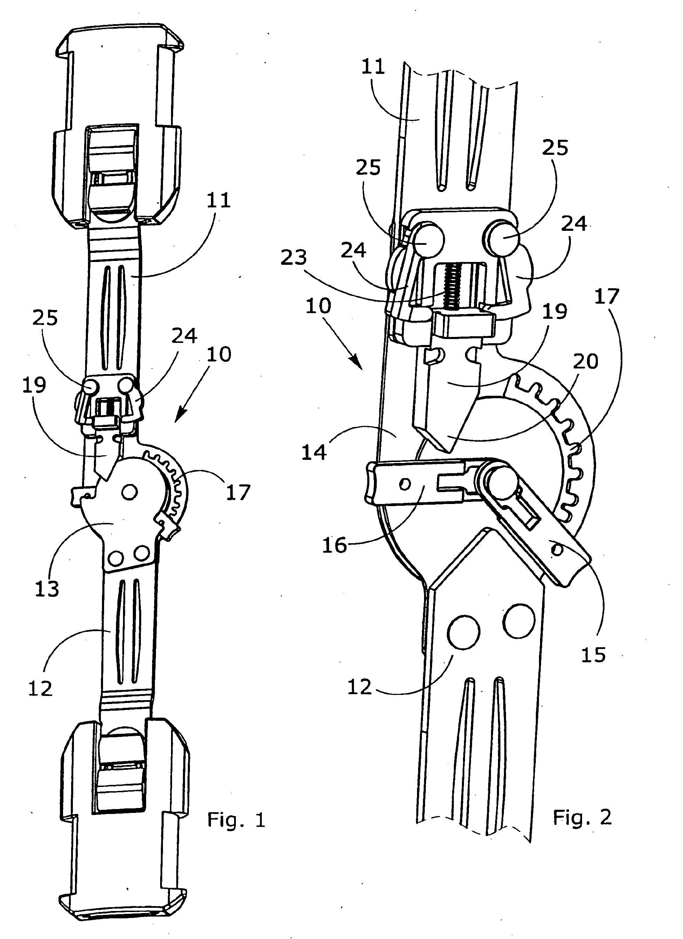

- figure 1 represents the schematic view of the quick-release restraining means according to the invention overall;
- figure 2 shows a schematic view of a detail of the coupling and quick-release unit, without the upper cover plate;

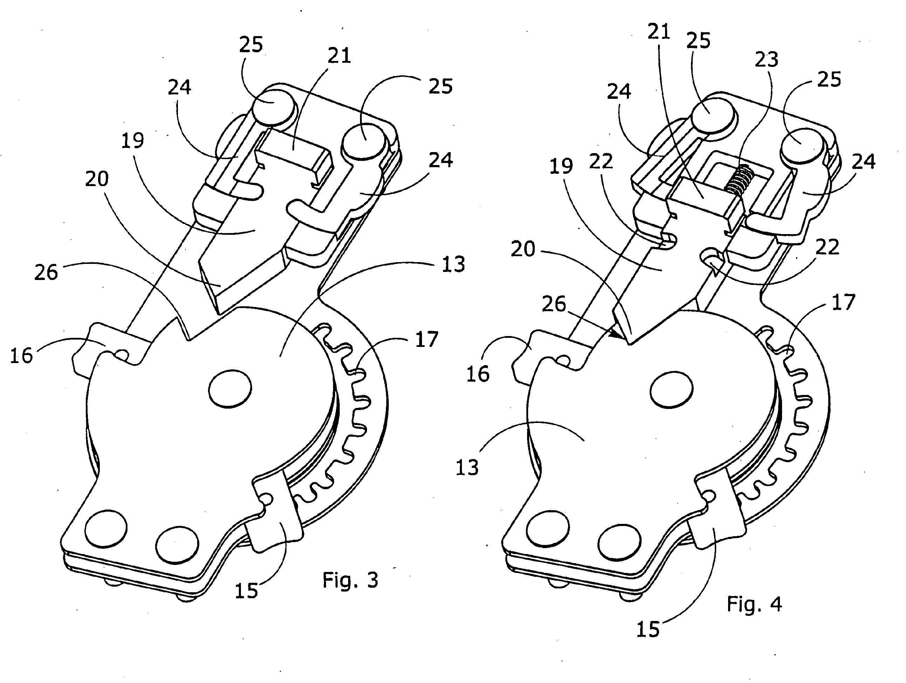

- figure 3 shows a schematic view of a detail of the restraining device with the quick-release means in the raised position and therefore in a released condition;
- figure 4 is a schematic view of the same detail as the previous figure, in this case with the restraining device in a blocked position;
- figure 5 represents an exploded schematic view of the restraining device according to the invention, showing all its components;
- figures 6 and 7 are schematic views of the restraining device not according to the invention as defined in claim 1;
- figures 8 and 9 represent schematic and detailed views of the restraining device according to the variation shown in figures 6 and 7.

### DESCRIPTION OF ONE EMBODIMENT OF THE INVENTION

The articulated joint for orthopedic devices or knee braces according to the invention is therefore equipped with a quick-release device which allows the knee brace to be maintained in a blocked position when used in weight-bearing conditions and when necessary to be released quickly by the user.

According to the invention, the articulated joint indicated overall with the reference number 10 is positioned between two uprights, that is the femoral upright 11 and the tibial upright 12.

The joint substantially consists of plates 13 connected to the tibial upright and plates 14 connected to the femoral upright.

The plates 13 and 14 are normally equipped with adjustable restraining means that allow the device to function in the classic way, that is with an angular excursion between one disc and the other limited within a range that gradually increases in relation to time.

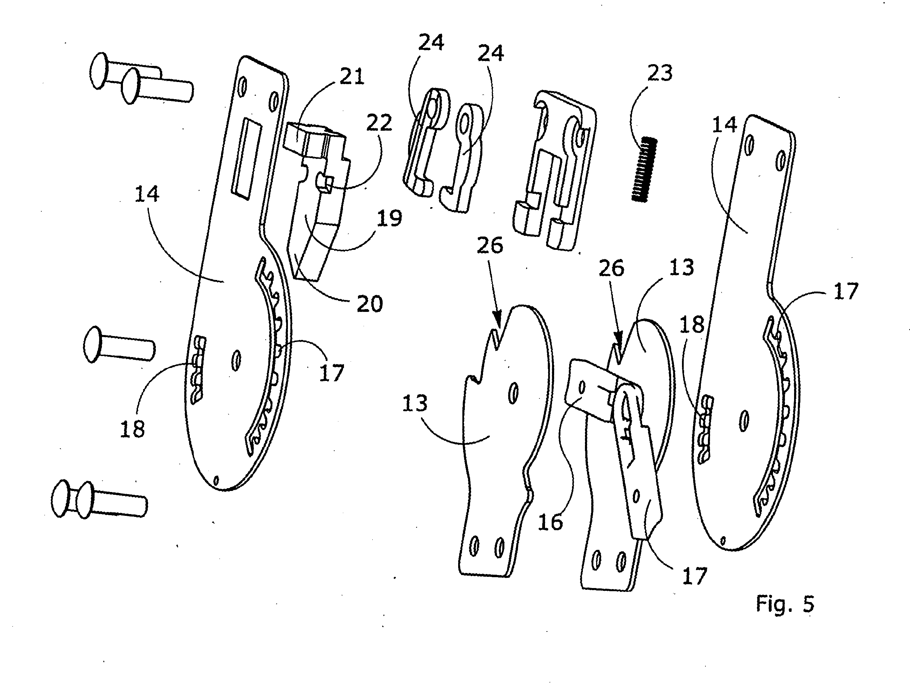

For this purpose, angularly adjustable elements, or registers, are used, indicated with 15 and 16, which make it possible to fix and to vary the flexion and extension angles of the knee joint and which engage in the toothed housings 17 and 18 better shown in figure 5.

According to the invention, the joint is equipped with quick-release means which substantially consist of a mobile cursor 19, which is substantially rectangular in shape and which terminates at its lower end with a wedge-shaped piece 20.

The upper part of the mobile cursor 19 is equipped with a tab 21, which can be gripped with the fingers, and with recesses 22 in the sides of the two opposite edges.

The mobile cursor 19 is positioned in opposition to a spring 23 located in a relative housing in such a way that it presses the cursor downwards acting against the upper side of the gripper tab 21.

The cursor 19 also works in conjunction at one end with restraining means consisting of a pair of hooks 24, hinged at 25 on the fixed part of the femoral upright and positioned so that they penetrate the recesses 22 in the cursor when it is in the raised position, while at the other end the wedge 20 is housed in the respective housing 26 in the plates 13, which are integral with the tibial upright.

The cursor can thus be moved from a blocked position in which the wedge 20 is inserted in and remains blocked in the respective housings 26 in the tibial plates, to a raised position released from the plates of the joint, allowing complete freedom of angular movement.

When the cursor is manually moved to the raised position, that is away from the plates, it is held in place by the two hook teeth 24 which are also spring-loaded towards each other and which penetrate the recesses 22.

To release the cursor 19 so that the wedge 20 can return to its position in the housing 26 in the plates 13, it is sufficient to disengage the two hooks 24 so that their ends are released from the recesses 22, allowing the cursor to return to the blocked position in opposition to the spring 23.

When the cursor returns to the blocked position with the wedge 20 inserted in the housing 26 of the plates 13, the articulated joint acts in the traditional way, that is it exerts an adjustable restraint on the knee joint allowing the brace to function in the classic way, that is with an angular excursion between one disc and the other limited within a range which gradually increases in relation to time. For this purpose it uses angularly adjustable elements 15 and 16 which make it possible to fix and vary the angle at which the knee joint flexes and extends and which engage in toothed housings 17 and 18.

It is clear that, once equipped with the quick-release restraining device, the knee brace articulated joint can be used in the most appropriate way and when necessary can be released from any angular restraint condition, allowing the user to make the necessary movements when no weight is being placed on the leg, for example when sitting down or getting into a car.

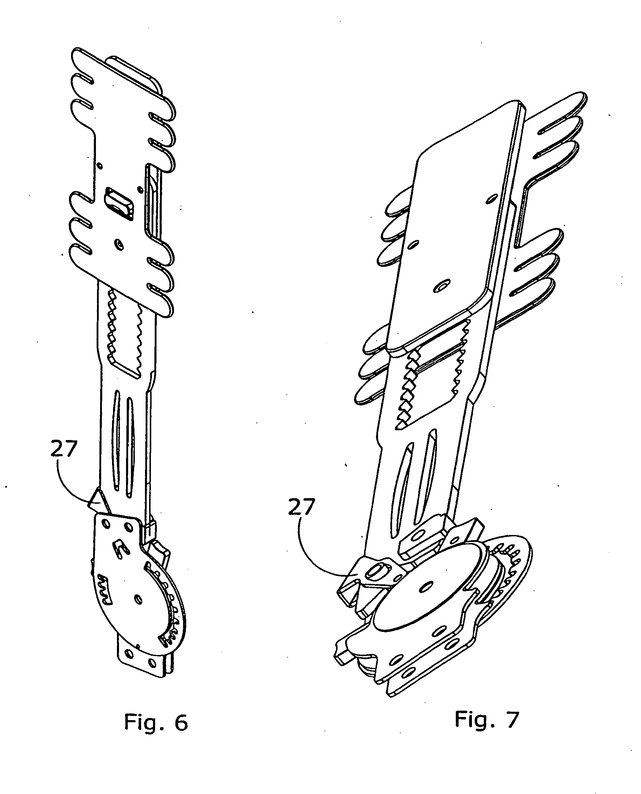

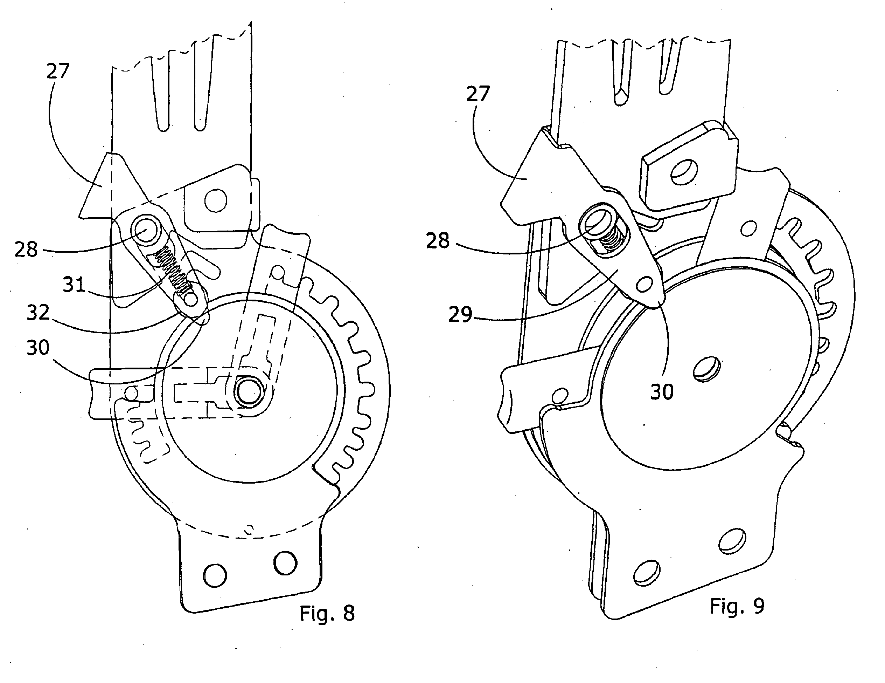

Figures 6 to 9 show the knee brace articulated joint according to another embodiment in which the cursor 19 is replaced by a front fork-like trigger 27.

The front trigger 27 is hinged at the point 28 on the upper upright and presents an appendix 30 with the same function as the wedge appendix 20 of the cursor 19.

The front trigger 27 also presents a central slot housing a spring 31 which acts against a wheel element 32.

The trigger is kept in a thrust condition by means of the spring 31, which thus determines the pressure of the wheel 32 and the appendix 30 against the respective housing in the lower upright. Similar to the previous case, the trigger 27 is moved with the fingers, overcoming the resistance of the spring 31, and in such a way that the trigger is held in a raised position by similar appendix blocking means 24.

The use of the device according to the invention, mainly for knee braces, can naturally be extended to any other type of orthopedic brace in which angular excursions are foreseen, that is to say for any brace which can be fitted indifferently to arm or leg joints.

The invention is described above with reference to a preferred embodiment.

## Claims

1. A knee brace articulated joint (10) consisting of plates (13) connected to the tibial upright (12) and plates (14) connected to the femoral upright (11), said plates (13, 14) being equipped with adjustable restraining means or registers (15, 16) which make it possible to vary the angular excursion between one disc and the other, limiting it within a predetermined range, said joint (10) being equipped with quick-release means that substantially consist of a mobile cursor element (19) that can be moved from a blocked position in which the devise is restrained angularly only by the registers (15, 16) to a raised position freed from the plates of the joint and allowing the joint to move angularly in complete freedom, **characterised in that** the mobile cursor element (19) presents a substantially rectangular shape which terminates at the flower end with a wedge-shaped appendix (20), while the top end is equipped with a tab (21) which can be gripped with the fingers and which presents recesses (22) in the sides of the two opposite edges, and **in that** the cursor (19) works in conjunction at one end with restraining means consisting of a pair of hooks (24), hinged (25) on the fixed part of the femoral upright and positioned so that they penetrate the recesses (22) in the cursor when it is in the raised position, while at the other end the wedge-shaped appendix (20) is housed in a respective housing (26) in the plates (13), which are integral with the tibial upright.

2. A knee brace articulated joint (10) according to claim 1, **characterised in that** the mobile cursor (19) is positioned in opposition to a spring (23) located in a relative housing in such a way that it presses the cursor downwards acting against the upper side of the gripper tab (21).

3. A knee brace articulated joint (10) according to any of the foregoing claims, **characterised in that** the cursor can be moved from a blocked position in which the wedge (20) is inserted in and remains blocked in the respective housings (26) in the tibial plates, to a raised position released from the plates of the joint, allowing complete freedom of angular movement.

4. A knee brace articulated joint (10) according to any of the foregoing claims, **characterised in that** when the cursor (19) is manually moved to the raised position, that is away from the plates, it is held in place by the two hook teeth (24) which are also spring-loaded towards each other and which penetrate the recesses (22).

5. A knee brace articulated joint (10) according to any of the foregoing claims, **characterised in that** the cursor (19) is implemended by a front fork-like trigger (27).

6. A knee brace articulated joint (10) according to claim 5, **characterised in that** said front trigger (27) is hinged at the point (28) on the upper upright and presents an appendix (30) with the same function as the wedge appendix (20) of the cursor (19).

7. A knee brace articulated joint (10) according to any one of claims 5 and 6, **characterised in that** said front trigger (27) presents a central slot housing a spring (31) which acts against a wheel element (32).

8. A knee brace articulated joint (10) according to claim 7, **characterised in that** said front trigger (27) is kept in a thrust condition by means of the spring (31), which thus determines the pressure of the wheel (32) and the appendix (30) against the respective housing in the flower upright.

## Patentansprüche

1. Artikuliertes Gelenk (10) für eine Kniestütze, mit Platten (13), die mit der Schienbeinstütze (12) verbunden sind, und mit Platten (14), die mit der Oberschenkelstütze (11) verbunden sind, wobei die Platten (13, 14) mit einstellbaren Rückhaltemitteln oder Registern (15, 16) ausgestattet sind, die es ermöglichen, dass die winkelmäßige Auslenkung zwischen der einen Scheibe und der anderen Scheibe variiert wird und diese innerhalb eines bestimmten Bereichs begrenzt ist, wobei das Gelenk (10) mit Schnellverschlussmitteln ausgestattet ist, die im Wesentlichen aus einem mobilen Zeigerelement (19) bestehen, welches aus einer verriegelten Stellung, in der die Vorrichtung winkelmäßig nur durch die Register (15, 16) gehalten ist, in eine angehobene Stellung verlagert werden kann, in der es von den Platten des Gelenks frei kommt und es dem Gelenk erlaubt ist, sich winkelmäßig vollständig frei zu bewegen, **dadurch gekennzeichnet, dass**
das mobile Zeigerelement (19) eine im Wesentlichen rechteckige Gestalt aufweist, die am unteren Ende mit einem keilförmigen Fortsatz (20) endet, während das obere Ende mit einem Halter (21) versehen ist, der mit den Fingern gegriffen werden kann und der Einschnitte (22) an den Kanten der beiden gegenüberliegenden Seiten aufweist, und dass
der Zeiger (19) an einem Ende mit den Rückhaltemitteln zusammenwirkt, die aus einem Paar von Haken (24) bestehen, die an dem feststehenden Abschnitt der Oberschenkelstütze angelenkt (25) sind und die so positioniert sind, dass sie in die Einschnitte (22) des Zeigers eindringen, wenn sich dieser in der angehobenen Position befindet, während an dem anderen Ende der keilförmige Fortsatz (20) in einem zugehörigem Gehäuse (26) in den Platten (13) aufgenommen ist, die integral mit der Schienbeinstütze ausgebildet sind.

2. Artikuliertes Gelenk (10) für eine Kniestütze nach Anspruch 1, **dadurch gekennzeichnet, dass** der mobile Zeiger (19) gegenüberliegend einer Feder (23) positioniert ist, die in einem zugehörigen Gehäuse so angeordnet ist, dass sie den Zeiger nach unten drückt, gegen die Oberseite des (Greif-) Halters (21) wirkend.

3. Artikuliertes Gelenk (10) für eine Kniestütze nach einem der vorherigen Ansprüche, **dadurch gekennzeichnet, dass** der Zeiger von einer verriegelten Position, in der der Keil (20) in den zugehörigen Gehäusen (26) der Schienbeinplatten eingeführt ist und dort verbleibt, in eine angehobene Position verlagert werden kann, von den Platten des Gelenks freigegeben, so dass die winkelmäßige Bewegung vollständig frei ist.

4. Artikuliertes Gelenk (10) für eine Kniestütze nach einem der vorherigen Ansprüche, **dadurch gekennzeichnet, dass**, wenn der Zeiger (19) manuell in die angehobene Position bewegt wird, d.h. weg von den Platten, er dort durch zwei Hakenzähne (24) in Position gehalten wird, die ebenfalls in Richtung aufeinander zu federbelastet sind, und die in die Vertiefungen (22) eindringen.

5. Artikuliertes Gelenk (10) für eine Kniestütze nach einem der vorherigen Ansprüche, **dadurch gekennzeichnet, dass** der Zeiger (19) durch einen gabelartigen vorderen Auslöser (27) ausgebildet ist.

6. Artikuliertes Gelenk (10) für eine Kniestütze nach Anspruch 5, **dadurch gekennzeichnet, dass** dieser vordere Auslöser (27) an dem Punkt (28) an der oberen Stütze angelenkt ist und und einen Fortsatz (30) darstellt, der die gleiche Funktion hat wie der keilförmige Fortsatz(20) des Zeigers (19).

7. Artikuliertes Gelenk (10) für eine Kniestütze nach einem der vorherigen Ansprüche 5 oder 6, **dadurch gekennzeichnet, dass** der vordere Auslöser (27) einen mittigen Schlitz hat, in der eine Feder (31) aufgenommen ist, die gegen ein Scheibenelement (32) wirkt.

8. Artikuliertes Gelenk (10) für eine Kniestütze nach Anspruch 7, **dadurch gekennzeichnet, dass** dieser vordere Auslöser (27) durch die Feder (31) in einem Schubzustand gehalten ist, wodurch wiederum der Druck der Scheibe (32) und des Fortsatzes (30) gegen das jeweilige Gehäuse an der unteren Stütze bestimmt ist.

## Revendications

1. Joint (10) articulé de genouillère consistant en des platines (13) connectées au montant tibial (12) et des platines (14) connectées au montant fémoral (11), lesdites platines (13, 14) étant équipées avec des moyens de restriction ou des registres ajustables (15, 16) qui rendent possible la variation de l'excursion angulaire entre un disque et l'autre, la limitant dans un intervalle prédéterminé, ledit joint (10) étant équipé de moyens de relâchement rapides qui consistent substantiellement en un élément de curseur mobile (19) qui peut être déplacé d'une position de blocage dans laquelle le dispositif est restreint angulairement seulement par les registres (15, 16) vers une position levée libérée des platines du joint et permettant au joint de bouger angulairement de manière complètement libre, **caractérisé en ce que** l'élément de curseur mobile (19) présente une forme substantiellement rectangulaire qui se termine à une extrémité basse par un appendice en forme de coin (20), tandis que l'extrémité supérieure est équipée d'une languette (21) pouvant être attrapée avec les doigts et qui présente des évidements (22) sur les faces de deux bords opposés, et **en ce que** le curseur (19) travaille en conjonction à une extrémité avec les moyens de restriction qui consistent en une paire de crochets (24) articulés (25) sur la partie fixée du montant fémoral et positionnés de sorte à pénétrer les évidements (22) dans le curseur quand il est en position levée, tandis qu'à l'autre extrémité l'appendice (20) en forme de coin est logé dans un logement (26) correspondant dans les platines (13), qui sont intégrées au montant tibial.

2. Joint (10) articulé de genouillère selon la revendication 1, **caractérisé en ce que** le curseur mobile (19) est positionné en opposition à un ressort (23) localisé dans un logement correspondant de telle sorte qu'il pousse le curseur vers le bas en agissant contre la face supérieure de la languette (21) d'accroche.

3. Joint (10) articulé de genouillère selon l'une quelconque des revendications précédentes, **caractérisé en ce que** le curseur peut être déplacé d'une position bloquée dans laquelle le coin (20) est inséré et reste bloqué dans les logements (26) correspondants dans les platines tibiales, à une position levée relâchée des platines du joint, permettant une complète liberté de mouvement angulaire.

4. Joint (10) articulé de genouillère selon l'une quelconque des revendications précédentes, **caractérisé en ce que** lorsque le curseur (19) est manuellement déplacé vers la position levée, qui est éloignée des platines, il est maintenu en place par les dents (24) des deux crochets qui sont aussi sollicités élastiquement l'un en direction de l'autre et qui pénètrent dans les évidements (22).

5. Joint (10) articulé de genouillère selon l'une quelconque des revendications précédentes, **caractérisé en ce que** le curseur (19) est implémenté par un déclencheur avant dentelé (27).

6. Joint (10) articulé de genouillère selon la revendication 5, **caractérisé en ce que** le déclencheur avant (27) est articulé au point (28) sur le montant supérieur et présente un appendice (30) avec la même fonction que l'appendice en coin du curseur (19).

7. Joint (10) articulé de genouillère selon l'une quelconque des revendications 5 et 6, **caractérisé en ce que** le déclencheur avant (27) présente une fente centrale logeant un ressort (31) qui agit contre un élément de roue (32)

8. Joint (10) articulé de genouillère selon la revendication 7, **caractérisé en ce que** le déclencheur avant (27) est maintenu dans un état de poussée aux moyens du ressort (31), qui détermine ainsi la pression de la roue (32) et de l'appendice (30) contre le logement correspondant dans le montant inférieur.
